# EUROPEAN PATENT SPECIFICATION

(11) **EP 4 260 071 B1**
(45) Date of publication and mention of the grant of the patent: **05.03.2025**
(21) Application number: 21854830.3
(22) Date of filing: 10.12.2021
(51) Int. Cl.: G01N 33/68

(54) **METHODS FOR DETERMINING THE RELATIVE DISTRIBUTION OF GLUCURONIDATION, IDURONIDATION, AND GALACTURONIDATION OF POLYPEPTIDES**
VERFAHREN ZUR BESTIMMUNG DER RELATIVEN VERTEILUNG VON GLUCURONIDIERUNG, IDURONIDIERUNG UND GALACTURONIDIERUNG VON POLYPEPTIDEN
PROCÉDÉS DE DÉTERMINATION DE LA DISTRIBUTION RELATIVE DE LA GLUCURONIDATION, DE L'IDURONIDATION ET DE LA GALACTURONIDATION DE POLYPEPTIDES

(30) Priority: 11.12.2020 US 202063124320 P
(43) Date of publication of application: 18.10.2023
(73) Proprietor: GENENTECH, INC., South San Francisco, CA 94080 (US)
(72) Inventor: YANG, Yi, South San Francisco, CA 94080-4990 (US)
(74) Representative: Brodbeck, Michel
(86) International application number: PCT/US2021/062843
(87) International publication number: WO 2022/125922

(56) References cited:
- US-A1- 2019 234 964
- NILSSON JONAS ET AL: "Characterization of Glycan Structures of Chondroitin Sulfate-Glycopeptides Facilitated by Sodium Ion-Pairing and Positive Mode LC-MS/MS", JOURNAL OF THE AMERICAN SOCIETY FOR MASS SPECTROMETRY, ELSEVIER SCIENCE INC, US, vol. 28, no. 2, 21 November 2016 (2016-11-21), pages 229 - 241, XP036132438, ISSN: 1044-0305, [retrieved on 20161121], DOI: 10.1007/S13361-016-1539-1

## Description

### FIELD OF INVENTION

The present description relates to methods for determining the relative distribution of glucuronidation, iduronidation, and galacturonidation of polypeptides. Specifically, the present description relates to stereoselective liquid chromatography-mass spectrometry (LC-MS) methods that achieve simultaneous separation and relative quantitation of glucuronidation, iduronidation, and galacturonidation of polypeptides.

### BACKGROUND

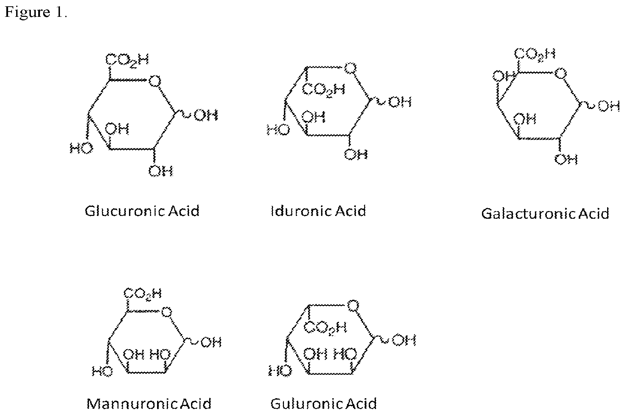

Glycuronidation is a post-translational polypeptide modification resulting from covalent attachment of a glycuronic acid (also known as uronic acid) to a primary amine group, such as the side chain of a lysine residue. There are five naturally occurring glycuronic acids: glucuronic acid, iduronic acid, galacturonic acid, mannuronic acid, and guluronic acid (Figure 1). In recombinant polypeptides produced by mammalian cells, e.g., CHO cells, as well as bacterial cells, e.g., E. coli, modification of primary amines by glucuronic acid, iduronic acid, galacturonic acid can occur. Modifications by mannuronic acid and guluronic acid, in contrast, are not expected in recombinantly expressed polypeptides since these two glycuronic acids are found in brown algea or in certain types of bacteria that are not commonly used for the production of recombinant polypeptides.

The modification of polypeptides by glucuronic acid, iduronic acid, or galacturonic acid has the potential to impact the safety and/or efficacy of such recombinantly-expressed products. For example, modification of lysine residues within complementarity-determining regions of monoclonal antibody drugs by glucuronic acid could interfere with target binding and thereby cause a loss in antibody potency. Alternatively, modification of lysine residues within complementarity-determining regions by iduronic acid has the potential to cause off-target binding due to the unique conformational flexibility of iduronic acid. In addition, because the modification of lysine residues by iduronic acid or galacturonic acid has not been observed on endogenous human polypeptides, iduronidation and galacturonidation of recombinantly-expressed polypeptides could potentially form a neoepitope, which may pose an immunogenic risk. To ensure the safety and efficacy of recombinantly-expressed polypeptides, it is important to determine relative distribution of glucuronidation, iduronidation, and/or galacturonidation.

Jonas Nilsson et al., (Journal of the American society for mass spectrometry, Vol. 28, No. 2, pages 229 - 241, 21 November 2016) describe a liquid chromatography-tandem mass spectrometry (LC-MS/MS) method allowing the characterization of glycopeptides originating from protease digests of glycoproteins.

US 2019/234964 provides a method for identifying glucuronylation of a protein by deglycosylating the protein and treating the deglycosylated protein with an enzyme and subjecting the resulting peptides to reverse phase liquid chromatography/mass spectroscopy analysis.

### SUMMARY OF THE INVENTION

The present invention relates to a method for determining the relative distribution of glucuronidation, iduronidation, and galacturonidation of a population of polypeptides which comprises:
a) contacting the population of polypeptides with a protease to generate peptides;
b) contacting the peptides to a chromatographic support comprising a hydrophobic stationary phase and a positive surface charge;
c) contacting the chromatographic support with a weak acid mobile phase gradient to separate the peptides;
d) analyzing the separated peptides to determine the relative distribution of glucuronidation, iduronidation, and galacturonidation of the peptides.

In certain embodiments, the protease is trypsin, lys-C, Glu-C, or Asp-N.

In certain embodiments, the chromatographic support comprises a support material with a positively charged surface modifier.

In certain embodiments, the hydrophobic stationary phase is linked to the surface modifier.

In certain embodiments, the hydrophobic stationary phase comprises an alkyl, alkenyl, alkynyl or aryl functional group.

In certain embodiments, the hydrophobic stationary phase comprises a C18 functional group.

In certain embodiments, the separated peptides are analyzed by using mass spectrometry.

In certain embodiments, the methods described herein comprise the identification of glucuronidated, iduronidated, and/or galacturonidated peptides.

In certain embodiments, the peptides are identified by their respective chromatographic retention time and mass spectra.

In certain embodiments, the methods described herein comprise the relative distribution of glucuronidation, iduronidation, and/or galacturonidation being determined by integrating the respective peaks of liquid chromatograms corresponding to the separated peptides as monitored by UV absorbance.

In certain embodiments, the relative distribution of glucuronidation, iduronidation, and/or galacturonidation is determined by integrating the respective peaks of ion chromatograms extracted from the mass spectrometry analysis.

In certain embodiments, the weak acid employed in the context of the methods described herein is formic acid or acetic acid.

In certain embodiments, the acidic mobile phase employed in the methods described herein is at a pH where greater than 50% of the carboxylic acid moieties associated with glucuronidation, iduronidation, or galacturonidation are in a deprotonated state.

In certain embodiments, the weak acid mobile phase gradient used in the context of the methods described herein has a pH>3.

In certain embodiments, the weak acid mobile phase gradient has a pKa value of about 4.5.

In certain embodiments, the mass spectrometry employed in the context of the methods described herein is electrospray ionization tandem mass spectrometry (ESI-MS/MS).

In certain embodiments, the polypeptide analyzed by the methods described herein is an antibody.

### BRIEF DESCRIPTION OF THE DRAWINGS

**Figure 1** depicts the structures of the naturally occurring glycuronic acids.
**Figure 2** depicts extracted ion chromatograms showing separation of glucuronidated (glua) and iduronidated (idoa) peptides. Antibody A was modified through forced glucuronidation/iduronidation. Samples were then digested by trypsin and analyzed by LC-MS/MS using a CSH C18 column. The IdoA and GluA peptides were eluted at 15.07 and 15.38 minute, respectively.
**Figure 3** depicts extracted ion chromatograms of the galacturonidated (glaa) peptide. Antibody A was modified through forced galacturonidation. Samples were then digested by trypsin and analyzed by LC-MS/MS using a CSH^{™} C18 column. The GlaA peptide was eluted at 15.16 minute.
**Figure 4** depicts the lack of stereoselective separation of candidate peptides on a BEH^{™} C18 column.

### DETAILED DESCRIPTION

The following Detailed Description, which is given by way of example, is not intended to limit the presently disclosed subject matter to specific embodiments described and may be understood in conjunction with the accompanying drawings.

The present description relates to methods for determining the relative distribution of glucuronidation, iduronidation, and/or galacturonidation of polypeptides. Specifically, the present description relates to stereoselective LC-MS methods that achieve simultaneous separation and relative quantitation of glucuronidation, iduronidation, and/or galacturonidation of polypeptides.

### 1. Definitions

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference, and the inclusion of such definitions herein should not necessarily be construed to represent a substantial difference over what is generally understood in the art.

The terms "comprise(s)," "include(s)," "having," "has," "can," "contain(s)," and variants thereof, as used herein, are intended to be open-ended transitional phrases, terms or words that do not preclude the possibility of additional acts or structures. The singular forms "a," "an" and "the" include plural references unless the context clearly dictates otherwise. The present disclosure also contemplates other embodiments "comprising," "consisting of" and "consisting essentially of," the embodiments or elements presented herein, whether explicitly set forth or not.

As used herein, the term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

As used herein, "polypeptide" refers generally to peptides and proteins having more than about ten amino acids. The polypeptides can be homologous to the host cell, or preferably, can be exogenous, meaning that they are heterologous, *i.e.,* foreign, to the host cell being utilized, such as a human protein produced by a Chinese hamster ovary cell, or a yeast polypeptide produced by a mammalian cell. In certain embodiments, mammalian polypeptides (polypeptides that were originally derived from a mammalian organism) are used, more preferably those which are directly secreted into the medium.

The term "protein" is meant to refer to a sequence of amino acids for which the chain length is sufficient to produce the higher levels of tertiary and/or quaternary structure. This is to distinguish from "peptides" or other small molecular weight drugs that do not have such structure. Typically, the protein herein will have a molecular weight of at least about 15-20 kD, preferably at least about 20 kD. Examples of proteins encompassed within the definition herein include all mammalian proteins, in particular, therapeutic and diagnostic proteins, such as therapeutic and diagnostic antibodies, and, in general proteins that contain one or more disulfide bonds, including multi-chain polypeptides comprising one or more inter- and/or intrachain disulfide bonds.

The term "antibody" is used herein in the broadest sense and encompasses various antibody structures including, but not limited to, monoclonal antibodies, polyclonal antibodies, monospecific antibodies (e.g., antibodies consisting of a single heavy chain sequence and a single light chain sequence, including multimers of such pairings), multispecific antibodies (e.g., bispecific antibodies), and antibody fragments so long as they exhibit the desired antigen-binding activity.

### 2. Methods for Determining the Relative Distribution gf Glucuronidation, Iduronidation, and Galacturonidation of a Polypeptide

In certain embodiments, the methods for relative distribution and/or identification of glucuronidation, iduronidation, and/or galacturonidation of polypeptides described herein comprise: a) contacting a polypeptide with a protease to generate peptides; b) contacting the peptides to a chromatographic support comprising a hydrophobic stationary phase and a positive surface charge; c) contacting the chromatographic support with a weak acid mobile phase gradient to separate the peptides; and d) analyzing the separated peptides to determine the relative distribution of glucuronidation, iduronidation, and/or galacturonidation of the peptides.

The methods outlined herein provide for the direct determination of the relative distribution of glucuronidation, iduronidation, and/or galacturonidation of polypeptides. These methods contrast with other approaches for investigating the glucuronidation, iduronidation, and/or galacturonidation of polypeptides. For example, approaches that measure the level of glucuronic acid in cell culture fluids do not necessarily correlate with the level of glucuronidation of polypeptides produced by such cultures, let alone provide any information concerning the distribution or identity of the modified amino acids of such polypeptides. The presently described approach also differs from methods requiring the release of glycuronic acids from peptides in the detection process, e.g., Lin et al., Eur. J Biochem, 106:341-351 (1980) and Spiro, JBC, 252(15): 5424-5430 (1977), as such approaches do not allow for the direct identification of the distribution or location of modified amino acids.

In certain embodiments, the protease is an aspartic, cysteine, metallo-, serine or threonine protease. Exemplary proteases useful in connection with the instant disclosure are known in the art, e.g., Verhamme et al. Proteases: Pivot Points in Functional Proteomics, Methods Mol Biol. 2019; 1871: 313-392, which is incorporated by reference in its entirety. In certain embodiments, the protease is trypsin. In certain embodiments, the protease is lys-C. In certain embodiments, the protease is Glu-C. In certain embodiments, the protease is Asp-N.

In certain embodiments, the chromatographic support comprises a support material with a positively charged surface modifier. In certain embodiments, the surface modifier can be attached to a chromatographic support base material via derivatization, coating, and/or crosslinking, thereby imparting the chemical character of the surface modifier to the base material of the chromatographic support. Exemplary surface modifiers are identified in U.S. Patent No. 7,223,473, which is hereby incorporated by reference in it is entirety. In certain embodiments, the surface modifier may be an organotrihalosilane, such as octyltrichlorosilane or octadecyltrichlorosilane. In certain embodiments, the chromatographic support comprises a hydrophobic stationary phase. In certain embodiments the hydrophobic stationary phase is linked, covalently or non-covalently, to the surface modifier. For example, but not by way of limitation, the surface modifier can comprise one or more alkyl, alkenyl, alkynyl, aryl functional groups as the hydrophobic stationary phase. In certain embodiments, the functional groups include, but are not limited to C₁-C₃₀ alkyl, such as octyl (C₈), octadecyl (C₁₈), and triacontyl (C₃₀); alkaryl, e.g., C₁-C₄-phenyl groups. The chromatographic support can be a Charged Surface Hybrid (CSH^{™}) stationary phase. The chromatographic support can be a CSH^{™} C18 stationary phase.

In certain embodiments, the acidic mobile phase is at a pH where greater than 50% of the carboxylic acid moieties associated with glucuronidation, iduronidation, and/or galacturonidation are in a deprotonated state. In certain embodiments, the weak acid is formic acid or acetic acid. In certain embodiments, the weak acid mobile phase gradient has pH>3. In certain embodiments, the weak acid mobile phase gradient has a pKa value of about 4.5.

In certain embodiments, the separated peptides are analyzed by using mass spectrometry. In certain embodiments, the mass spectrometry is electrospray ionization tandem mass spectrometry (ESI-MS/MS).

In certain embodiments, the methods of presently disclosed subject matter further comprise the identification of glucuronidated, iduronidated, and/or galacturonidated peptides. In certain embodiments, the peptides are identified by their respective chromatographic retention time and/or mass spectra. In certain embodiments, the relative distribution of glucuronidation, iduronidation, and/or galacturonidation is determined by integrating the respective peaks of liquid chromatograms corresponding to the separated peptides as monitored by UV absorbance. In certain embodiments, the relative distribution of glucuronidation, iduronidation, and/or galacturonidation is determined by integrating the respective peaks of ion chromatograms extracted from the mass spectrometry analysis.

In certain embodiments, the polypeptide is an antibody. The antibody can be a monoclonal antibody. The antibody can be a multispecific antibody, e.g., a bispecific or a trispecific antibody.

### EXAMPLES

### Example 1: Assay for Determining the Relative Distribution of Glucuronidation, Iduronidation, and Galacturonidation of Polypeptides

Antibody A polypeptides were digested using a protease (such as trypsin, lys-C, Glu-C, or Asp-N). The resulted peptides were separated by using a CSH^{™} C18 column. The stereoselective separation was based on a charge-charge interaction between the negative charge from glycuronic acid and the positive surface charge from the stationary phases and a hydrophobic interaction between the glycuronidated peptides and the hydrophobic stationary phase. A mobile phase gradient using weak acids (such as formic or acetic acid, but not trifluoroacetic acid) in the mobile phases was employed to achieve separation of glucuronidated, iduronidated, and galacturonidated peptides. The weak acids were selected, since, at pH> 3, majority of the carboxylic groups (pKa=~4.5) from the glycuronic acid moiety is negatively charged, which promotes a stereoselective charge-charge interaction between the glycuronic acid moiety and the positively charged stationary phase.

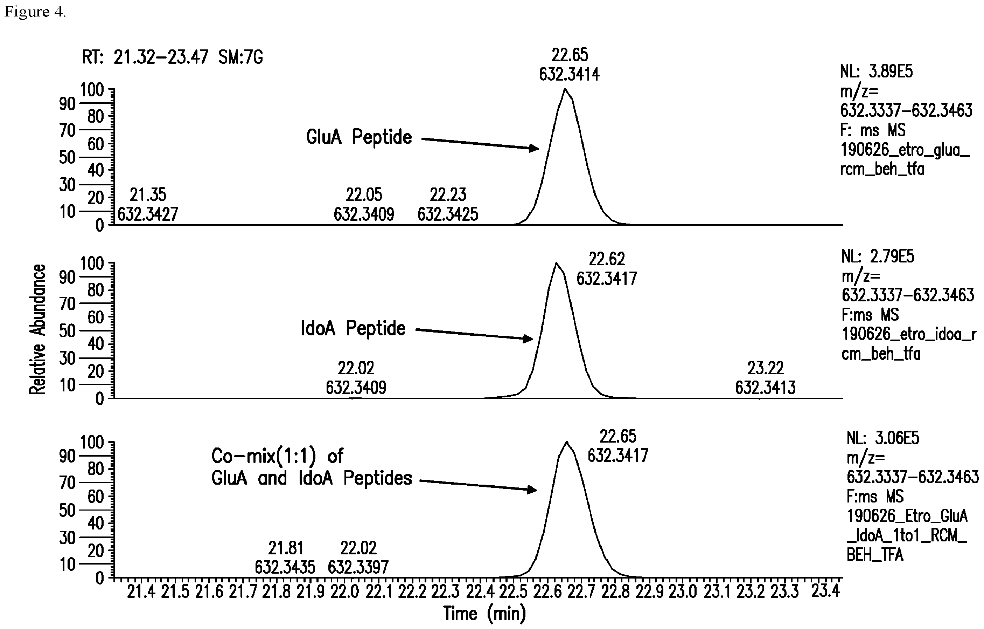

Antibody A polypeptides were digested using a protease (such as trypsin, lys-C, Glu-C, or Asp-N). The resulted peptides were analyzed by using a BEH^{™} C18 column, whose stationary phase is not positively charged. A mobile phase gradient using trifluoroacetic acid in the mobile phases (pH <3) was employed, where trifluoroacetic acid is a commonly used ion pair reagent to improve peak shape for improved chromatographic separation. As shown in Figure 4, the stereoselective separation cannot be achieved on the BEH^{™} C18 column.

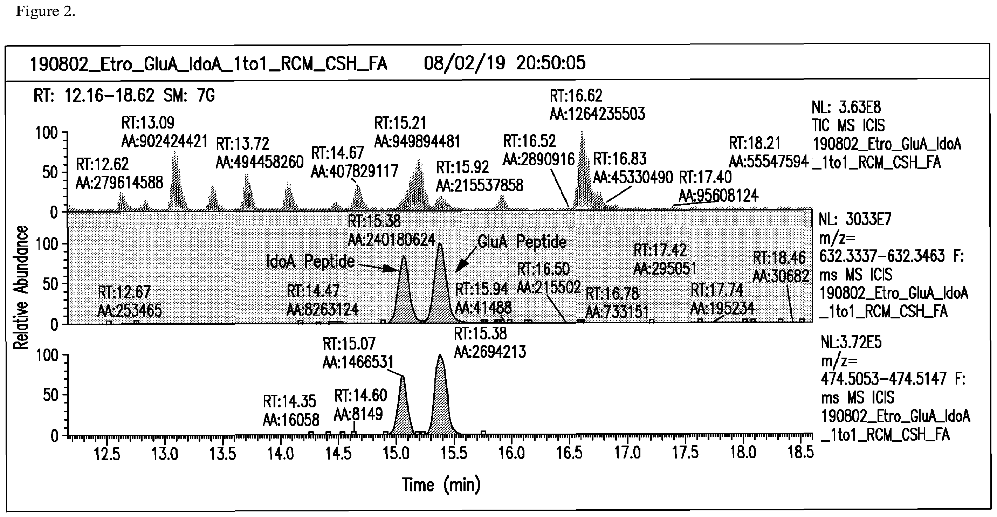

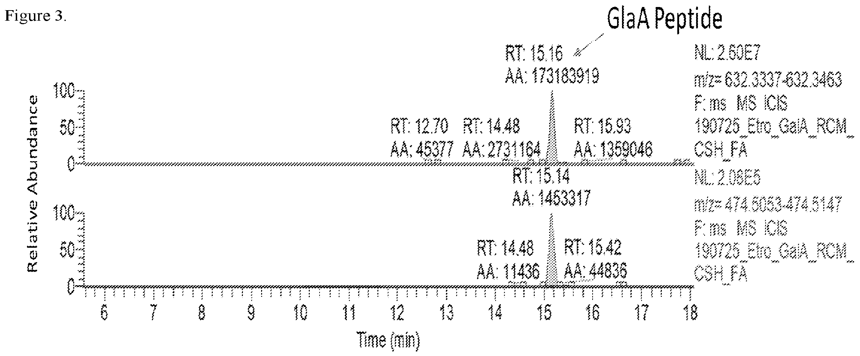

The eluted peptides were subsequently analyzed by ESI-MS/MS. The identity of glucuronidated, iduronidated, and galacturonidated peptides was confirmed by their respective retention time from the chromatographic separation (Figures 2 and 3) and by their mass spectra collected from the ESI-MS/MS analysis. The relative distribution of glucuronidation, iduronidation, and galacturonidation was determined by integrating the respective peaks on the liquid chromatograms (monitored by UV absorbance) or on the extracted ion chromatograms (based on the molecular mass, Figures 2 and 3).

## Claims

1. A method for determining the relative distribution of glucuronidation, iduronidation, and galacturonidation of a population of polypeptides, the method comprising:
a) contacting the population of polypeptides with a protease to generate peptides;
b) contacting the peptides to a chromatographic support comprising a hydrophobic stationary phase and a positive surface charge;
c) contacting the chromatographic support with a weak acid mobile phase gradient to separate the peptides;
d) analyzing the separated peptides to determine the relative distribution of glucuronidation, iduronidation, and/or galacturonidation of the peptides.

2. The method of claim 1, wherein the protease is trypsin, lys-C, Glu-C, or Asp-N.

3. The method of claim 1, wherein the chromatographic support comprises a support material with a positively charged surface modifier.

4. The method of claim 3, wherein the hydrophobic stationary phase is linked to the surface modifier.

5. The method claim 4, wherein the hydrophobic stationary phase comprises an alkyl, alkenyl, alkynyl or aryl functional group.

6. The method of claim 5, wherein the hydrophobic stationary phase comprises a C18 functional group.

7. The method of claim 1, wherein the separated peptides are analyzed by using mass spectrometry.

8. The method of claim 1, comprising the identification of glucuronidated, iduronidated, and galacturonidated peptides.

9. The method of claim 7, wherein the peptides are identified by their respective chromatographic retention time and mass spectra.

10. The method of claim 1, wherein the relative distribution of glucuronidation, iduronidation, and galacturonidation is determined by integrating the respective peaks of liquid chromatograms corresponding to the separated peptides as monitored by UV absorbance.

11. The method of claim 1, wherein the relative distribution of glucuronidation, iduronidation, and galacturonidation is determined by integrating the respective peaks of ion chromatograms extracted from the mass spectrometry analysis.

12. The method of claim 1, wherein the weak acid is formic acid or acetic acid.

13. The method of claim 1, wherein the acidic mobile phase is at a pH where greater than 50% of the carboxylic acid moieties associated with glucuronidation, iduronidation, or galacturonidation are in a deprotonated state.

14. The method of claim 1, wherein the weak acid mobile phase gradient has pH>3.

15. The method of claim 1, wherein the weak acid mobile phase gradient has a pKa value of about 4.5.

16. The method of claim 1, wherein the mass spectrometry is electrospray ionization tandem mass spectrometry (ESI-MS/MS).

17. The method of claim 1, wherein the polypeptide is an antibody.

## Patentansprüche

1. Verfahren zum Bestimmen der relativen Verteilung von Glucuronidierung, Iduronidierung und Galacturonidierung einer Population von Polypeptiden, wobei das Verfahren Folgendes umfasst:
a) Inkontaktbringen der Population von Polypeptiden mit einer Protease, um Peptide zu erzeugen;
b) Inkontaktbringen der Peptide mit einem chromatographischen Träger, der eine hydrophobe stationäre Phase und eine positive Oberflächenladung umfasst;
c) Inkontaktbringen des chromatographischen Trägers mit einem Gradienten von schwachen Säuren in der mobilen Phase, um die Peptide abzuspalten;
d) Analysieren der abgespaltenen Peptide, um die relative Verteilung von Glucuronidierung, Iduronidierung und/oder Galacturonidierung der Peptide zu bestimmen.

2. Verfahren nach Anspruch 1, wobei die Protease Trypsin, Lys-C, Glu-C oder Asp-N ist.

3. Verfahren nach Anspruch 1, wobei der chromatographische Träger ein Trägermaterial mit einem positiv geladenen Oberflächenmodifikator umfasst.

4. Verfahren nach Anspruch 3, wobei die hydrophobe stationäre Phase mit dem Oberflächenmodifikator verknüpft ist.

5. Verfahren nach Anspruch 4, wobei die hydrophobe stationäre Phase eine funktionelle Alkyl-, Alkenyl-, Alkinyl- oder Aryl-Gruppe umfasst.

6. Verfahren nach Anspruch 5, wobei die hydrophobe stationäre Phase eine funktionelle C18-Gruppe umfasst.

7. Verfahren nach Anspruch 1, wobei die abgespaltenen Peptide unter Anwendung von Massenspektrometrie analysiert werden.

8. Verfahren nach Anspruch 1, umfassend die Identifikation von glucuronidierten, iduronidierten und galacturonidierten Peptiden.

9. Verfahren nach Anspruch 7, wobei die Peptide anhand ihrer jeweiligen chromatographischen Retentionszeit und Massenspektren identifiziert werden.

10. Verfahren nach Anspruch 1, wobei die relative Verteilung von Glucuronidierung, Iduronidierung und Galacturonidierung durch Integrieren der jeweiligen Peaks von Flüssigchromatogrammen bestimmt wird, die den abgespaltenen Peptiden entsprechen, überwacht anhand von UV-Absorption.

11. Verfahren nach Anspruch 1, wobei die relative Verteilung von Glucuronidierung, Iduronidierung und Galacturonidierung durch Integrieren der jeweiligen Peaks von Ionenchromatogrammen, die aus der Massenspektrometrieanalyse extrahiert werden, bestimmt wird.

12. Verfahren nach Anspruch 1, wobei die schwache Säure Ameisensäure oder Essigsäure ist.

13. Verfahren nach Anspruch 1, wobei die saure mobile Phase einen pH aufweist, bei dem mehr als 50 % der Carbonsäureeinheiten in Verbindung mit Glucuronidierung, Iduronidierung oder Galacturonidierung in einem deprotonierten Zustand vorliegen.

14. Verfahren nach Anspruch 1, wobei der Gradient von schwachen Säuren in der mobilen Phase einen pH > 3 aufweist.

15. Verfahren nach Anspruch 1, wobei der Gradient von schwachen Säuren in der mobilen Phase einen pKa-Wert von etwa 4,5 aufweist.

16. Verfahren nach Anspruch 1, wobei die Massenspektrometrie Elektrospray-Ionisation-Tandem-Massenspektrometrie (ESI-MS/MS) ist.

17. Verfahren nach Anspruch 1, wobei das Polypeptid ein Antikörper ist.

## Revendications

1. Procédé de détermination de la distribution relative de la glucuronidation, de l'iduronidation et de la galacturonidation d'une population de polypeptides, le procédé comprenant :
a) la mise en contact de la population de polypeptides avec une protéase pour générer des peptides ;
b) la mise en contact des peptides avec un support chromatographique comprenant une phase stationnaire hydrophobe et une charge de surface positive ;
c) la mise en contact du support chromatographique avec un gradient de phase mobile acide faible pour séparer les peptides ;
d) l'analyse des peptides séparés pour déterminer la distribution relative de la glucuronidation, de l'iduronidation et/ou de la galacturonidation des peptides.

2. Procédé selon la revendication 1, dans lequel la protéase est la trypsine, la lys-C, la Glu-C ou l'Asp-N.

3. Procédé selon la revendication 1, dans lequel le support chromatographique comprend un matériau de support avec un modificateur de surface chargé positivement.

4. Procédé selon la revendication 3, dans lequel la phase stationnaire hydrophobe est liée au modificateur de surface.

5. Procédé selon la revendication 4, dans lequel la phase stationnaire hydrophobe comprend un groupe fonctionnel alkyle, alcényle, alcynyle ou aryle.

6. Procédé selon la revendication 5, dans lequel la phase stationnaire hydrophobe comprend un groupe fonctionnel en C18.

7. Procédé selon la revendication 1, dans lequel les peptides séparés sont analysés en utilisant la spectrométrie de masse.

8. Procédé selon la revendication 1, comprenant l'identification de peptides glucuronidés, iduronidés et galacturonidés.

9. Procédé selon la revendication 7, dans lequel les peptides sont identifiés par leurs temps de rétention chromatographique et spectres de masse respectifs.

10. Procédé selon la revendication 1, dans lequel la distribution relative de la glucuronidation, de l'iduronidation et de la galacturonidation est déterminée par l'intégration des pics respectifs de chromatogrammes liquides correspondant aux peptides séparés tels que surveillés par absorbance UV.

11. Procédé selon la revendication 1, dans lequel la distribution relative de la glucuronidation, de l'iduronidation et de la galacturonidation est déterminée par l'intégration des pics respectifs de chromatogrammes ioniques extraits de l'analyse de la spectrométrie de masse.

12. Procédé selon la revendication 1, dans lequel l'acide faible est l'acide formique ou l'acide acétique.

13. Procédé selon la revendication 1, dans lequel la phase mobile acide est à un pH où plus de 50 % des fractions acides carboxyliques associées à la glucuronidation, à l'iduronidation ou à la galacturonidation sont dans un état déprotoné.

14. Procédé selon la revendication 1, dans lequel le gradient de phase mobile acide faible a un pH>3.

15. Procédé selon la revendication 1, dans lequel le gradient de phase mobile acide faible a une valeur de pKa d'environ 4,5.

16. Procédé selon la revendication 1, dans lequel la spectrométrie de masse est une spectrométrie de masse en tandem avec une ionisation par électrospray (ESI-MS/MS).

17. Procédé selon la revendication 1, dans lequel le polypeptide est un anticorps.
